# EUROPEAN PATENT SPECIFICATION

(11) **EP 2 505 670 B1**
(45) Date of publication and mention of the grant of the patent: **12.07.2017**
(21) Application number: 12161906.8
(22) Date of filing: 28.03.2012
(51) Int. Cl.: C12Q 1/68

(54) **Method for the identification of SNPs**
Verfahren zur Identifizierung von SNPs
Méthode pour l'identification de SNPs

(30) Priority: 28.03.2011 IT TO20110271
(43) Date of publication of application: 03.10.2012
(73) Proprietor: Genefast S.r.l., 40053 Bazzano (BO) (IT)
(72) Inventor: Gentilini, Fabio, 47121 Forlí (FC) (IT); Turba, Maria Elena, 47121 Forlí (FC) (IT)
(74) Representative: Robba, Pierpaolo

(56) References cited:
- EP-A1- 1 686 190
- US-A1- 2003 148 301
- US-A1- 2010 286 143
- US-B1- 6 207 425
- LUNDBERG ET AL: "A rapid one-tube PCR method for simultaneously differentiating homozygotes and heterozygotes of the Sp1 binding site polymorphism in collagen type Ialpha1", MOLECULAR AND CELLULAR PROBES, ACADEMIC PRESS, LONDON, GB, vol. 21, no. 3, 16 March 2007 (2007-03-16) , pages 239-241, XP005924005, ISSN: 0890-8508, DOI: DOI:10.1016/J.MCP.2006.10.007
- MILBURY C A ET AL: "COLD-PCR: Improving the sensitivity of molecular diagnostics assays", EXPERT REVIEW OF MOLECULAR DIAGNOSTICS 2011 EXPERT REVIEWS LTD. GBR LNKD- DOI:10.1586/ERM.10.115, vol. 11, no. 2, 10 March 2011 (2011-03-10) , pages 159-169, XP002649700, ISSN: 1473-7159
- JIN LI ET AL: "Coamplification at Lower Denaturation Temperature-PCR Increases Mutation-Detection Selectivity of TaqMan-Based Real-Time PCR", CLINICAL CHEMISTRY, AMERICAN ASSOCIATION FOR CLINICAL CHEMISTRY, WASHINGTON, DC, vol. 55, no. 4, 1 April 2009 (2009-04-01), pages 748-756, XP002638590, ISSN: 0009-9147, DOI: DOI:10.1373/CLINCHEM.2008.113381
- CARVALHO ET AL: "Study of disease-relevant polymorphisms in the TLR4 and TLR9 genes: A novel method applied to the analysis of the Portuguese population", MOLECULAR AND CELLULAR PROBES, ACADEMIC PRESS, LONDON, GB, vol. 21, no. 4, 11 May 2007 (2007-05-11), pages 316-320, XP022077437, ISSN: 0890-8508, DOI: DOI:10.1016/J.MCP.2007.03.005

## Description

### Field of the invention

The present invention relates to a method of identifying nucleotide sequence variations, in particular single nucleotide genetic polymorphisms.

### State of the art

Genetic polymorphisms are nucleotide sequence variations that may occur anywhere in the genome of an organism. Among the most common types of polymorphisms, there are the single nucleotide polymorphisms, commonly referred to in the art with the acronym SNP, which will be used hereinafter throughout the description. A single nucleotide polymorphism is characterised by the presence of different nucleotide bases in the same locus of different individuals or organisms.

Polymorphisms are an expression of the genetic variability that makes every individual or organism different from another one of the same species. Independently of their biological role, SNPs are of essential importance for disciplines exploiting genetics: they are used in microbiology in order to distinguish microorganism strains belonging to the same species, as well as in evolutional genetics in order to understand phylogeny, or they are used as genetic markers in studies concerning both monogenic hereditary disease linkage and quantitative trait linkage (QTL). Linkage analyses by means of QTL are also the basis of all genetic investigations in zootechnics and agriculture for a genetic selection based on traits of economic interest, such as weight increase, yield increase or resistance to diseases.

The enormous applicative potentialities of SNP genotyping, i.e. of the identification of the allele variation of a polymorphism, have led over time to the development of several analysis methods. The methods may be sequence-specific or non sequence-specific. The first mentioned methods are of particular interest, since they allow identifying the precise locus and the exact nature of a SNP, and not only detecting its presence. The invention concerns a method of this kind.

Many of the sequence-specific methods used at present for SNP genotyping comprise amplifying the SNP locus by means of polymerase chain reaction, commonly referred to in the art with the acronym PCR, which will be used hereinafter throughout the description. Such methods are particularly interesting from both the technical and the commercial standpoint, since they are simple, robust and relatively cheap, especially when the set of primers being used allows differentiating among the different alleles by means of a single reaction.

EP 1686190 discloses an allele-specific amplification method that can be carried out within a single reaction tube. The method uses forward primers with different lengths, having therefore different characteristics of double-stranded DNA separation temperature (melting temperature, Tm), and a common reverse primer. The discrimination among the different alleles is based on the analysis of the melting temperatures of the PCR products (amplicons). Differentiation among Tm characteristics is obtained by means of nonspecific sequences (tails) with different lengths at the 5' end of the forward primers. In order to result in a good discrimination, the difference in the tail lengths must be high and this entail a relatively long length for the longer tail (10 to 26 bases in the examples). This makes the reaction less efficient, facilitates the possibility of creating stable secondary structures of the primer and the occurrence of dimers of the primer, which potentially can make the interpretation uncertain or the assay design more difficult.

Another method that can be carried out within a single reaction tube is disclosed in US 6,207,425 and in the paper "Overlapping PCR for Bidirectional PCR Amplification of Specific Alleles: A Rapid One-Tube Method for Simultaneously Differentiating Homozygotes and Heterozygotes", by Q. Liu et al., GENOME RESEARCH, 1997, no. 7, pages 389 - 398.

This method uses four oligonucleotide primers, namely two inner, allele-specific primers and two outer primers, for amplifying two alleles. The inner primers are designed with their 3' ends in correspondence of the polymorphism, each on a respective one of the DNA strands and in mutually opposite directions, and they have at their 5' ends a non-complementary oligonucleotide tail characterised by a high G (guanine) and C (cytosine) content, such as to limit the overpriming phenomenon and thus to increase specificity. Also the outer primers are provided on both strands, are anti-parallel and are designed at different distances from the inner primers. A DNA fragment subjected to PCR by using such a set of primers generates two or three PCR products depending on whether homozygosis or heterozygosis is present. The amplicon generated by the outer primers (product "wt+mut") is always present. The second and/or the third amplicons (products "wt" and "mut") are generated in the presence of the wild-type allele and/or the mutant allele, respectively, and are due to the combination of the outer and inner primers complementary to the sequence of the respective allele. Products "wt" and "mut" have a respective specific length and, thus, the presence of wild-type or mutant alleles can be detected after agarose gel electrophoresis.

This method has some drawbacks. A first drawback is that the presence of the band produced by the outer primers (product "wt+mut") makes the PCR interpretation complex and, substantially, limits the visualisation possibilities of the assay to the only visualisation on agarose gel, while hindering at the same time the possibility of interpretation by means of real time PCR in closed tube. Moreover, the band produced by the outer primers, due to the manner in which it is generated, contains sequences of both product "wt" and product "mut". Since the presence of such a band is unavoidable, the assay product can be neither adapted to finalised methods based on multiple PCR, nor used for array hybridisation assays, since the presence of the amplicon "wt+mut" would subtract the hybridisation probes. Furthermore, the design using oligonucleotide tails of the above type often makes the specific amplification of one allele, or even the amplification of all alleles, scarcely efficient. When the efficiency of amplification of one allele is unfavourably affected, it is possible that the corresponding amplicon is not generated, thus creating conditions in which a wrong genotyping can readily be attributed. When, on the contrary, the efficiency of amplification of both alleles is inadequate, the assay in the whole is scarcely efficient and demands high amounts of DNA.

### Summary of the invention

It is therefore an object of the present invention to provide a method of identifying natural or artificially induced nucleotide sequence variations, which is effective, accurate, versatile and cheap and obviates the drawbacks of the prior art. The present invention is reflected in the appended claims. According to the present invention, a PCR reaction with the simultaneous use of two different primer pairs is used, as disclosed in US 6,207,425. Hence, each primer pair comprises an allele-specific primer having its 3' end moiety located at the polymorphous locus. The allele-specific primers are complementary to the anti-parallel strands and have an anti-parallel mutual orientation. The second primer of each pair is designed so as to generate, with the corresponding allele-specific primer located on the anti-parallel strand, a product with such a size or such melting temperature characteristics that the product itself can be readily differentiated.

According to a preferred feature of the invention, the PCR reaction includes a melting step occurring at a temperature lower than the critical melting temperature Tc of the product generated by the priming with the combination of both outer primers, which has a length corresponding to the sum of the lengths of the products obtained in the presence of wild-type alleles or mutant alleles in homozygosis.

The provision of a such a step at reduced temperature intrinsically and wholly prevents the occurrence of the product generated by the outer primers, so that only the products due to the presence of either the wild-type allele or the mutant allele (product "wt" and product "mut", respectively) are present in the reaction tube at the end of the PCR reaction. Consequently, the product can be readily and unambiguously genotyped by both the analysis by visualisation through agarose gel electrophoresis and the analysis of the melting curve in case of real time PCR.

Otherwise stated, there are used a pair of primers amplifying the wild-type reference DNA and a pair of primers amplifying the mutant DNA. A primer of the pair amplifying the wild-type DNA (in particular the forward primer) and a primer of the pair amplifying the mutant DNA (in particular the reverse primer) have the polymorphism, for the wild-type and the mutant variation, respectively, at the 3' end moiety and are characterised in that they are complementary and anti-parallel to the DNA antisense strand and sense strand, respectively. The other primer of the pair amplifying the mutant DNA and the other primer of the pair amplifying the wild-type DNA are complementary and anti-parallel to the DNA antisense strand and sense strand, respectively. The different combinations of the four primers may give rise to three different products. Two products are due to the priming with the combination of both primers of the pair amplifying the wild-type DNA and with the combination of both primers of the pair amplifying the mutant DNA, respectively, and are formed in the presence of the wild-type allele and the mutant allele, respectively. A third product is due to the priming with the combination of the forward primer of the pair amplifying the mutant DNA and the reverse primer of the pair amplifying the wild-type DNA, and it can be generated whichever allele is present. The three different products have different sizes, different melting temperatures Tm and different critical temperatures Tc (where "critical temperature" denotes the melting temperature immediately below which the efficiency of the PCR reaction abruptly decreases), such that the third product has a size, a melting temperature and a critical temperature higher than both other products. By making the melting step occur at a temperature lower than the Tc of the third product and higher than the Tc characteristic of the other two products, only one or two of the three potential products can be generated, depending on the presence of the nucleotide variation, and such product(s) can be detected or differentiated by size after electrophoretic separation on agarose gel, or by determining the specific melting temperature (by means of the analysis of the melting curve in case of real time PCR).

PCR with low-temperature melting (Cold PCR) is known per se, for instance from WO 2009/017784, where however it is used for causing enrichment of PCR products containing unknown variations with reduced critical temperature, in order to make detection of such variations easier in subsequent sequencing analyses, and not for preventing amplification of certain products of the PCR reaction.

Other references disclosing Cold PCR are "MILBURY C A ET AL: 'COLD-PCR: Improving the sensitivity of molecular diagnostics assays', EXPERT REVIEW OF MOLECULAR DIAGNOSTICS 2011 EXPERT REVIEWS LTD. GBR LNKO-DOI:10.1586/ERM.10.115, vol.11, no.2, 10 March 2011 (2011-03-10), pages 159-169, and "JIN LI ET AL: 'Coamplification at Lower Denaturation Temperature-PCR Increases Mutation-Detection Selectivity of TaqMan-Based Real-Time PCR', CLINICAL CHEMISTRY, AMERICAN ASSOCIATION FOR CLINICAL CHEMISTRY, WASHINGTON, DC, vol. 55, no.4, 1 April 2009 (2009-04-01), pages 748-756. Also these documents disclose use of Cold PCR for the enrichment of PCR products, and in no way they suggest the use for preventing the amplification of an unwanted reaction product containing both polymorphisms, without resulting in the enrichment of one variation over the other.

The absence of the third product, i.e. product "wt+mut" due to the outer primers, makes the method particularly flexible, since it allows using the method also in multiplex processes and in hybridisation matrix assays which, as said, are hindered just by that product.

Preferably, the inner primers are hairpin primers. Hairpin design is a practical, cheap and versatile method and, using constant thermodynamic variables, allows obtaining a high and foreseeable efficiency for the PCR reaction.

Preferably, the invention is applied in genetic tests. A preferred application is the detection of the polymorphism responsible for the von Willebrand genetic disease of type I in dogs.

### Brief description of the drawings

Other particularities and features of the invention will become apparent from the following description, with reference to the accompanying drawings, in which:
- Figs. 1A to 1C are schematic representations showing the hybridisation positions and the orientations of the primers used according to the invention, as well as the amplification reaction products, for both copies of a DNA fragment for a wild-type homozygote organism, a heterozygote organism and a mutant homozygote organism, respectively;
- Figs. 2A to 2C show DNA sequences containing the polymorphism to be genotyped, for the wild-type homozygote organism, the heterozygote organism and the mutant homozygote organism, respectively, in a preferred application of the invention;
- Fig. 3 shows the setting up of the assay, in the preferred application of the invention, with visualisation of the PCR products of samples with known genotype;
- Fig. 4 shows the result of the genotyping with visualisation of the PCR products for field samples;
- Fig. 5 shows the melting curves obtained as a result of the genotyping of samples with known genotype by means of real-time PCR; and
- Figs. 6A to 6C show the melting curves of Fig. 5 separately for the heterozygote organism, the mutant homozygote organism and the wild-type homozygote organism.

### Detailed description of preferred embodiments

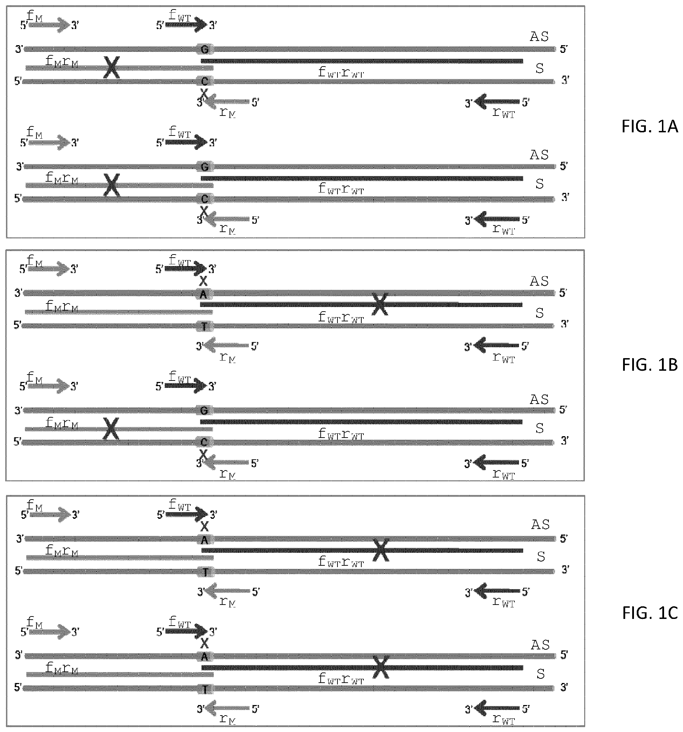

Figs. 1A to 1C schematically show the principle of the invention, with reference to an exemplary application in which the polymorphism to be identified has a base G or a base A in one of the DNA strands and, consequently, a base C or a base T in the other strand. The Figures show both strands of both copies of a DNA fragment for the wild-type homozygote organism (Fig. 1A), the heterozygote organism (Fig. 1B) and the mutant homozygote organism (Fig. 1C), respectively.

The invention is based on the simultaneous use of two different pairs of primers, shown by arrows f_{WT}, r_{WT}, f_{M}, r_{M} in Figs. 1A to 1C, which primers are added to a conventional PCR mixture.

Each primer pair comprises a first primer (f_{WT} and r_{M}, respectively, hereinafter referred to as inner primer or allele-specific primer), which has the polymorphism at the 3' end moiety, and a second primer (f_{M} and r_{WT}, respectively, hereinafter referred to as outer primer or non allele-specific primer).

Primer f_{WT} is specific for the wild-type allele (i.e. it has the base typical of the wild-type homozygote organism) and is complementary and anti-parallel to the negative or antisense DNA strand AS, and primer r_{M} is specific for the mutant allele (i.e. it has the base typical of the mutant homozygote organism) and is complementary and anti-parallel to the positive or sense DNA strand S. The anti-parallel orientation of the allele-specific primers makes the allele-specific nucleotide incorporation in a PCR amplification reaction take place mediated by a DNA polymerase in divergent direction, as disclosed in US 6,207,425.

As to the two other primers f_{M}, r_{WT}, one of them (f_{M}) is complementary and anti-parallel to the negative strand at a position downstream (i.e. in the direction towards the 3' end on the negative strand AS) the position of the allele-specific primer specific for the mutant allele, and the other (r_{WT}) is complementary and anti-parallel to the positive strand at a position downstream (i.e. in the direction towards the 3' end on the positive strand S) the position of the allele-specific primer specific for the wild-type allele.

Because of such a configuration, primers f_{WT}, r_{WT} may also be defined as the forward and reverse primer, respectively, for the wild-type allele and primers f_{M}, r_{M} as the forward and reverse primer, respectively, for the mutant allele.

Preferably, in order to increase the specificity of the allele-specific bonds of the inner primers, the allele-specific primers f_{WT}, r_{M} are modified by the addition, at the 5' end, of a variable number of nucleotides complementary and anti-parallel to the sequences at the 3' end, so that the oligonucleotide takes a hairpin shape. The use of hairpin primers in a genotyping method based on PCR amplification is disclosed for instance in WO 2004/061134.

Thanks to the use of hairpin primers, the annealing temperature of the primers for the PCR reaction can be increased, thereby increasing specificity, without having at the same time deleterious effects on the assay efficiency. To this aim, the optimal length of the primer without the nucleotide tail at its 5' end should be 18 to 25 nucleotides with a calculated melting temperature in the range 55°C to 60°C. In turn, the nucleotide tail at the 5' end would be optimal if it has a length in the range 5 to 7 nucleotides, with an enthalpy, calculated as -ΔG of the hairpin structure, in the range -4.2 to -7.0 under the following conditions: 25°C, [Na⁺] = 270 mM, [Mg⁺⁺] = 3 mM.

By using the primers described above, for each copy of the sequence, a product f_{WT}r_{WT} (product "wt") will be obtained in the presence of the wild-type allele and a product f_{M}r_{M} (product "mut") will be obtained in the presence of the mutant allele. The two products have different sizes, different melting temperatures Tm and different critical temperatures Tc. As known, the term "critical temperature" denotes the melting temperature at which the efficiency of the PCR reaction abruptly decreases, that is the temperature below which a specific PCR target is not amplified. Critical temperature Tc is proportional to, but lower than the respective melting temperature Tm.

The allele-specific primer that is not operating in a given situation is marked by "X" in Figs. 1A to 1C.

According to an advantageous feature of the present invention, the PCR reaction takes place by using, in the melting step, a temperature that is lower than the critical temperature Tc of the product due to outer primers f_{M} and r_{WT} (product "wt+mut" that, with the symbols used, is product f_{M}r_{WT}) and higher than the critical temperatures Tc of both f_{WT}r_{WT} and f_{M}r_{M}, respectively. Thanks to such a feature, formation of product f_{M}r_{WT} is substantially avoided and only one, or both, of the allele-specific PCR products f_{WT}r_{WT} and f_{M}r_{M} is or are obtained, as shown in Figs. 1A to 1C. The absence of the product due to the outer, non allele-specific primers allows obviating the drawbacks described above with reference to US 6,207,425. The product(s) obtained may be identified e.g. by size, after electrophoretic separation on agarose gel, or by determining the specific melting temperature (by means of the analysis of the melting curve in case of real time PCR).

Advantageously, according to the invention, a temperature lower by few tenths of degree (e.g. 0.1 to 0.5°C) than the temperature Tc of product f_{M}r_{WT} is used in the melting step.

Given a certain set of primers, determining the temperature to be used in the melting step in the reaction is not a problem for the skilled in the art. Indeed, product f_{M}r_{WT}, due to the intrinsic mechanism generating it, has a length, in terms of number of nucleotide bases, that is close to the sum of the lengths of the allele-specific products f_{WT}r_{WT} and f_{M}r_{M}, and therefore it has a Tc that, due to its nature, is higher than the Tc of the allele-specific products.

The application of the invention to the detection of the polymorphism responsible for the von Willebrand genetic disease of type I in dogs will now be described. The disease is autosomal and recessive and it is due to the substitution G > A, shown in Figs. 1A to 1C, in the last nucleotide of exon 43 of the von Willebrand factor. The mutation is expressed as c,7437G>A ENSCAFT00000024188. In particular, the substitution facilitates a cryptic splicing site located four base pairs (bp) upstream the normal site and determines a shift of the isoform reading frame generated by the alternative splicing, resulting in the formation of a truncated peptide of 119 AA. This disease affects several dog races, including Dobermann, Bernese, Coton de Tuler, Pinscher, Kerry blue terrier, Manchester terrier, Pembroke Welsh corgi, Poodle, Shetland sheepdog.

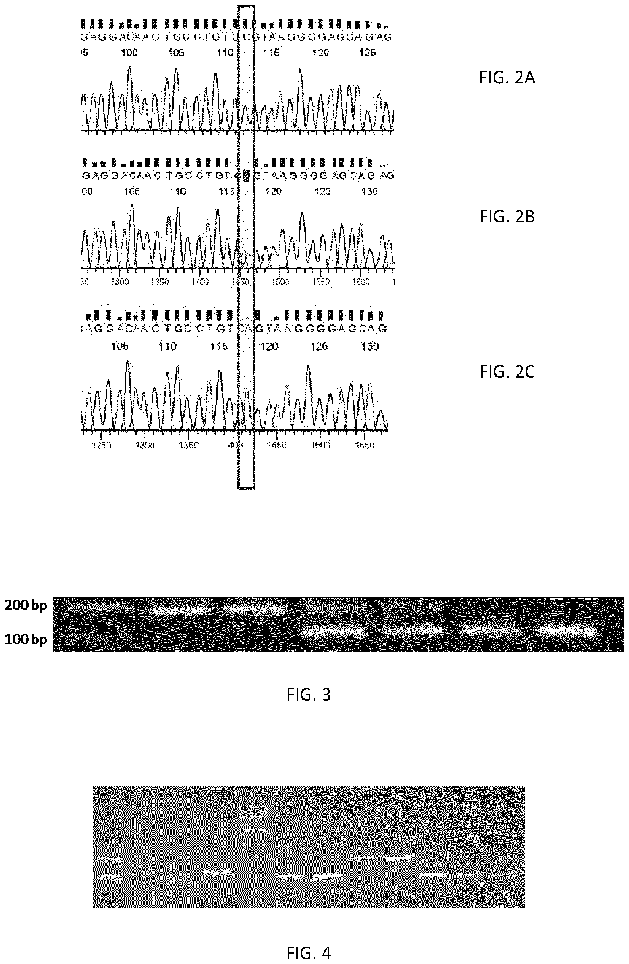

In the example described herein, there were used Dobermann subjects which were sound, heterozygote carrier and suffering from von Willebrand disease of type I, respectively. As shown also in Fig. 2, sound subjects have a nucleotide G in homozygosis, carrier subjects have nucleotides G and A in heterozygosis and subjects suffering from von Willebrand disease have a nucleotide A in homozygosis.

Genotyping of the locus responsible for the disease has been carried out by means of direct sequencing of PCR products including the locus, as described above. The two pairs of primers used have been designed by using the reference genomic sequences of von Willebrand factor in dogs and a specific software for designing the primers. Software products for this purpose are commercially available.

The primer sequences and their thermodynamic characteristics are reported in the following table:

| Primer | Sequence | r | product length (bp) |
|---|---|---|---|
| Wild-type forward (f_{WT}) | CGACAGTGTGAGGACAACTGCCTGTCG | 67.4 | 184 |
| Wild-type reverse (r_{WT}) | GCTGCCTTTCACCCAACCTC | 58.5 | |
| Mutant forward (f_{M}) | GGACTTGGAGGACTCTGTGATG | 57.5 | 101 |
| Mutant reverse (r_{M}) | AGTAAGGCCCCTCTGCTCCCCTTACT | 66.3 | |

The segments underlined in solid line at the left ends (5' ends) of the sequences of primers f_{WT}, r_{M} denote the tails that have been added to obtain the hairpin structure. The complementarity and the anti-parallelism between the tails and the sequence portions at the 3' end, underlined in dotted line, are clearly apparent.

Samples of purified genomic DNA taken from blood of such subjects, made non coagulable in K₃EDTA, have been subjected to PCR using the following reaction mixture:
- Buffer: 1x
- Mg⁺⁺: 2 mM
- dNTP (deoxyribonucleoside triphosphates): 250 mM each
- mutant forward primer: 400 nM
- mutant reverse primer: 300 nM
- wild-type forward primer: 200 nM
- wild-type reverse primer: 150 nM
- Taq polymerase Hot Start: 1 unit
- water for molecular biology: 23 µl
- genomic DNA: 2 µl

Under the PCR conditions reported above, the critical temperature Tc of product f_{M}r_{WT} generated by outer primers f_{M} and r_{WT}, which product would have a length of 233 bp, is 87.9°C.

In the exemplary application of the invention described herein, the amplification protocol carried out on a thermal cycler is as follows: initial melting for 3 min at 95°C, then 40 melting cycles at 87.7°C for 30 sec, annealing at 67°C for 15 sec and extension at 72°C for 15 sec.

The temperature at which the melting cycles take place is therefore lower by 0.2°C than the Tc of product f_{M}r_{WT}.

The PCR products visualised after dyeing with an intercalating dye and electrophoretic separation in agarose gel have the appearance shown in Figs. 3 and 4.

In particular, Fig. 3 shows the setting up of the assay with visualisation of the PCR products of samples with known genotype. Lane 1 shows the bands of the reference markers for the length (or the molecular weight) of the products; lanes 2 and 3 show the bands relevant to sound subjects (wild-type homozygotes), lanes 4 and 5 show the bands relevant to carrier subjects (heterozygotes), and lanes 6 and 7 show the bands relevant to ill subjects (mutant homozygotes).

Fig. 4 shows the results of the genotyping of field samples: lane 1 is relevant to a heterozygote subject; lanes 2 and 3 are relevant to two cases in which genotyping could not be carried out; lanes 4, 6, 7, 10, 11 and 12 are relevant to subjects suffering from the disease; lane 5 is relevant to a molecular weight marker; lanes 8 and 9 are relevant to sound subjects. The absence of product f_{M}r_{WT} is clearly apparent in Fig. 4.

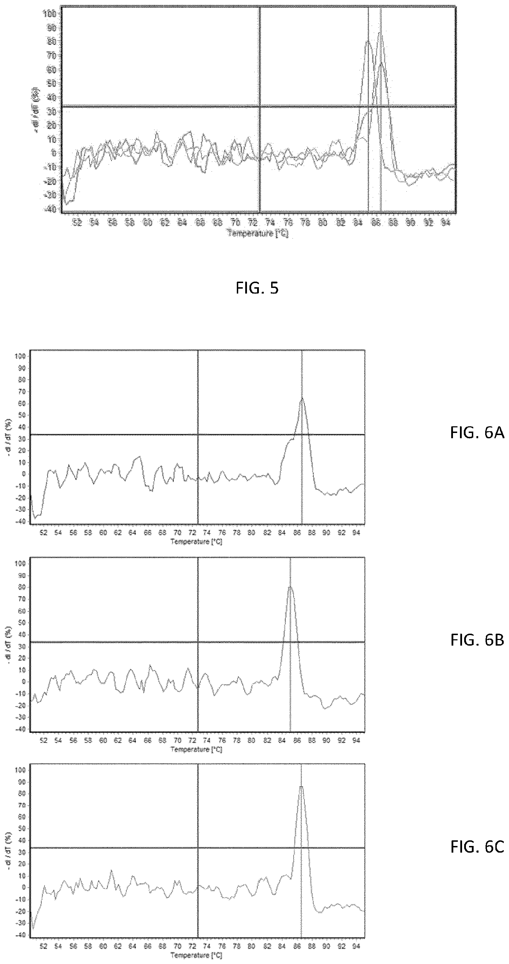

The same protocol carried out by means of real time PCR, again on samples with known genotype, originates melting curves (reporting the derivative of the fluorescence, in percentage, versus temperature) as shown in Fig. 5 (where the curves are superimposed) and in Figs. 6A to 6C. In the latter Figures, Fig. 6A relates to the heterozygote, Fig. 6B relates to the wild-type homozygote and Fig. 6C relates to the mutant homozygote.

It is clear that the above description has been given only by way of non-limiting example and that changes and modifications are possible without departing from the scope of the invention.

In particular, even though the invention has been described with reference to the identification of the alleles of a natural polymorphism in which a base substitution occurs, by suitably designing the primers the invention can also be used for identifying the alleles of an artificially obtained polymorphism, e.g. a polymorphism obtained after bisulphite reaction in DNA methylation analysis, as well as in case of natural or chemically induced polymorphism in which a base addition or deletion occurs.

Moreover, even though the use of hairpin primers has been described in detail, also other kinds of primers can be used, such as primers modified with locked nucleic acid (LNA™). In general, the primers can be those used in genotyping methods based on PCR with allele-specific primer extension.

Furthermore, even if the results of a detection in a homogeneous phase solution have been disclosed, detection can also take place on a solid support (arrays or chips).

## Claims

1. A method of identifying nucleotide sequence variations, comprising the steps of:
- amplifying a DNA sample, including the site of a variation to be identified, by means of a single polymerase chain reaction (PCR) with the simultaneous use of four different primers including:
- an inner primer (f_{WT}) specific for the wild-type allele, having a base complementary to the corresponding base of the wild-type allele at its 3' end and being complementary and anti-parallel to the negative or antisense DNA strand (AS);
- an outer primer (f_{M}) non allele specific, complementary and anti-parallel to a region in the negative strand or antisense strand (AS) at a position situated 3' of the (f_{WT}) primer, on the same strand;
- an inner primer (r_{M}), specific for the mutant allele, having a base complementary to the corresponding base of the mutant allele at its 3' end and being complementary and anti-parallel to the positive or sense DNA strand (S);
- an outer primer (r_{WT}) non allele specific, complementary and anti-parallel to a region in the positive strand or sense DNA strand (S) at a position situated 3' of the (r_{M}) primer on the same strand;
- obtaining for each copy of the sequence, a product (wt) in the presence of the wild-type allele and a product (mut) in the presence of the mutant allele wherein:
- the two products are differentiated by size, melting temperature (Tm) and critical temperature (Tc) - where "critical temperature" denotes the melting temperature below which a specific PCR target is not amplified;
- detecting the wild-type (wt) and mutant (mut) reaction products on the basis of said differentiating characteristics by means of electrophoretic separation on agarose gel, or by determining the specific melting temperature by means of the analysis of the melting curve in case of real time PCR,
**characterized in that** the method further comprises the step of:
- preventing the occurrence of the product generated by the two outer primers, by means of a melting step carried out at a temperature lower than the critical melting temperature (Tc) of the product generated by the priming with the combination of the two outer primers, and higher than the critical temperature (Tc) characteristic of the other two products generated by the (f_{WT}) and (r_{WT}) primers and by the (f_{M}) and (r_{M}) primers, so that only these two products (wt) and (mut) are present at the end of the PCR reaction.

2. The method as claimed in claim 1, wherein the temperature of said melting step is lower by some tenths of degree than the critical temperature of said product generated by the priming with the combination of both outer primers (f_{M}, r_{WT}).

3. The method as claimed in claim 1 or 2, wherein the primers are chosen out of the primers usually employed in PCR-based genotyping methods with allele-specific primer extension, in particular hairpin primers and primers modified with locked nucleic acid.

4. The method as claimed in claim 3, wherein the allele-specific primers are hairpin primers having, without the nucleotide tail originating the hairpin structure, a length in the range 18 to 25 nucleotides and a melting temperature Tm in the range 55°C to 60°C, and the nucleotide tail has a length in the range 5 to 7 nucleotides with an enthalpy, calculated as -ΔG of the hairpin structure, in the range -4.2 to - 7.0 at ambient temperature and in standard PCR buffer solutions.

5. The method as claimed in any preceding claim, wherein said detecting step is performed by electrophoretic separation on a gel or in a capillary tube or by the analysis of standard or high-resolution melting curves.

6. The method as claimed in any of claims 1 to 4, wherein said detecting step is performed on a solid support.

7. The method as claimed in any preceding claim, used for diagnostic genetic tests.

8. The method as claimed in claim 7, wherein the diagnostic tests are tests aimed at identifying the polymorphism responsible for the von Willebrand's genetic disease of type I in dogs.

9. The method as claimed in any of claims 1 to 6, wherein the inner primers are primers specific for the variations resulting from polymorphisms obtained after bisulphite reaction in DNA methylation analysis.

10. The method according to claim 8, wherein the four primers are designed by using the reference genomic sequences of von Willebrand factor in dogs and wherein the primer sequences and their thermodynamic characteristics are as follows:
| Primer | Sequence | r | product length (bp) |
|---|---|---|---|
| Wild-type forward (f_{WT}) | CGACAGTGTGAGGACAACTGCCTGTCG | 67.4 | 184 |
| Wild-type reverse (r_{WT}) | GCTGCCTTTCACCCAACCTC | 58.5 | |
| Mutant forward (f_{M}) | GGACTTGGAGGACTCTGTGATG | 57.5 | 101 |
| Mutant reverse (r_{M}) | AGTAAGGCCCCTCTGCTCCCCTTACT | 66.3 | |

11. The method according to claim 10, wherein the segments at the left 5' ends of the sequences of wild-type inner primer (f_{WT}) and mutant inner primer (r_{M}) are the tails added to obtain a hairpin structure.

12. The method according to claim 11, wherein the single polymerase chain reaction (PCR) is carried out by means of a reaction mixture comprising:
- Buffer: 1x
- Mg++: 2 mM
- dNTP (deoxyribonucleoside triphosphates): 250 mM each
- mutant forward primer: 400 nM
- mutant reverse primer: 300 nM
- wild-type forward primer: 200 nM
- wild-type reverse primer: 150 nM
- Taq polymerase Hot Start: 1 unit
- water for molecular biology: 23 µl
- genomic DNA: 2 µl.

13. The method according to claim 12, wherein the critical temperature (Tc) of the product (f_{M}r_{WT}) generated by outer primers (f_{M}, r_{WT}), having a length of 233 bp, is 87.9°C.

14. The method according to claim 12 or 13, wherein the amplification protocol is carried out on a thermal cycler by means of: initial melting for 3 min at 95°C, then 40 melting cycles at 87.7°C for 30 sec, annealing at 67°C for 15 sec and extension at 72°C for 15 sec.

15. The method according to claim 14, wherein the temperature at which the melting cycles take place is lower by 0.2°C than the Tc of product (f_{M}r_{WT}) generated by outer primers (f_{M}, r_{WT}).

## Patentansprüche

1. Verfahren zur Identifizierung von Variationen von Nukleotid-Sequenzen mit den Schritten von:
- Vervielfältigen einer DNA-Probe, welche den Ort der zu identifizierenden Variation enthält, mittels einer einzelnen Polymerase-Kettenreaktion (PCR) bei gleichzeitigem Einsatz von vier verschiedenen Primern mit:
- einem inneren Primer (f_{WT}), der für das Wild-Typ-Allel spezifisch ist und eine Basis aufweist, die zu der entsprechenden Basis des Wild-Typ-Allels an seinem 3'-Ende komplementär ist und komplementär sowie antiparallel zu dem negativen oder antisense DNA-Strang (AS) ist;
- einem äußeren Primer (f_{M}), der nicht allel-spezifisch, komplementär und antiparallel zu einem Abschnitt des negativen oder antisense Strangs (AS) an einer 3' gelegenen Position des (f_{WT}) Primers an demselben Strang ist;
- einem inneren Primer (r_{M}), der für das mutante Allel spezifisch ist, eine zur entsprechenden Basis des mutanten-Allels komplementäre Basis an seinem 3'-Ende aufweist und komplementär und antiparallel zu dem positiven oder sense DNA-Strang (S) ist;
- einem äußeren Primer (r_{WT}), der nicht allele-spezifisch, komplementär und antiparallel zu einem Abschnitt des positiven Strangs oder sense DNA-Strang (S) an einer 3' gelegenen Position des (r_{M}) Primers an dem selben Strang ist;
- Erhalten für jede Kopie der Sequenz ein Produkt (wt) bei Anwesenheit des Will-Typ-Allels und eines Produkts (mut) bei Anwesenheit des mutanten Allels, wobei:
- die beiden Produkte an ihrer Größe, Schmelztemperatur (Tm) und kritischen Temperatur (Tc) unterschieden werden - wobei "kritische Temperatur" die Schmelztemperatur angibt, unterhalb welcher ein spezifisches PCR-Ziel nicht vervielfältigt wird;
- Detektieren des Wild-Typ (wt) und mutanten (mut) Reaktionsprodukts auf der Basis der erwähnten unterscheidenden Merkmale mittels einer elektrophoretischen Trennung auf Agarosegel oder durch Ermitteln der spezifischen Schmelztemperatur mittels Analyse der Schmelzkurve im Fall von Echtzeit-PCR,
**dadurch gekennzeichnet, dass** das Verfahren zusätzlich den folgenden Schritt aufweist:
- Verhindern des Auftretens des Produkts, das von den beiden äußeren Primern erzeugt wird, mittels eines Schmelzschritts, der bei einer Temperatur durchgeführt wird, die kleiner als die kritische Schmelztemperatur (Tc) des Produkts, das durch das Vorbereiten mit der Kombination der beiden äußeren Primer erzeugt wurde, und größer als die kritische Temperatur (Tc) ist, die für die beiden anderen Produkte charakteristisch ist, die durch die (f_{WT}) und (r_{WT}) Primer sowie durch die (f_{M}) und (r_{M}) Primer erzeugt wurden, so dass nur diese beiden Produkte (wt) und (mut) am Ende der PCR-Reaktion vorliegen.

2. Verfahren nach Anspruch 1, wobei die Temperatur des Schmelzschritts einige Zehntel Grad niedriger als die kritische Temperatur des erwähnten Produkts ist, das durch das Vorbereiten mit der Kombination der beiden äußeren Primer (f_{M}, r_{WT}) erzeugt wurde.

3. Verfahren nach Anspruch 1 oder 2, wobei die Primer aus den Primern ausgewählt sind, die üblicherweise in PCR-basierten Genotypisierungs-Verfahren mit Allele-spezifischen Primer-Extensionen eingesetzt werden, insbesondere Haarnadel-Primern und mit gesperrter Nukleinsäure modifizierten Primern.

4. Verfahren nach Anspruch 3, wobei die Allele-spezifischen Primer Haarnadel-Primer sind, die ohne einen Nukleotidschwanz, der von der Haarnadelstruktur ausgeht, eine Länge im Bereich von 18 bis 25 Nukleotiden und eine Schmelztemperatur Tm im Bereich von 55 °C bis 60 °C haben, und deren Nukleotidschwanz eine Länge im Bereich von 5 bis 7 Nukleotiden mit einer Enthalpie, die als -ΔG der Haarnadelstruktur berechnet ist, im Bereich von -4,2 bis -7,0 bei Umgebungstemperatur und in Standart-PCR-Pufferlösungen hat.

5. Verfahren nach einem der vorstehenden Ansprüche, wobei der Detektionsschritt durch elektrophoretische Trennung auf einem Gel oder in einem Kapillarröhrchen oder durch die Analyse von Standard- oder hochaufgelösten Schmelzkurven durchgeführt wird.

6. Verfahren nach einem der Ansprüche 1 bis 4, wobei der Detektionsschritt auf einem festen Träger durchgeführt wird.

7. Verfahren nach einem der vorstehenden Ansprüche, eingesetzt für diagnostische Gentests.

8. Verfahren nach Anspruch 7, wobei die diagnostischen Tests Tests sind, die darauf abzielen, den Polymorphismus zu identifizieren, der für die von Willebrandsche Erbkrankheit des Typs I bei Hunden verantwortlich ist.

9. Verfahren nach einem der Ansprüche 1 bis 6, wobei die inneren Primer Primer sind, die spezifisch für Variationen sind, die aus Polymorphismus resultieren, der nach einer Bisulfit-Reaktion bei einer DNA-Methylierungsanalyse erhalten werden.

10. Verfahren nach Anspruch 8, wobei die vier Primer durch Einsatz der Referenz-Gensequenzen des von Willebrand Faktors in Hunden entwickelt werden, und wobei die Primersequenzen und ihre thermodynamischen Eigenschaften folgendermaßen sind:
| Primer | Sequenz | r | Produktlänge (bp) |
|---|---|---|---|
| Wild-Typ vorwärts (f_{WT}) | CGACAGTGTGAGGACAACTGCCTGTCG | 67,4 | 184 |
| Wild-Typ rückwärts (r_{WT}) | GCTGCCTTTCACCCAACCTC | 58,5 | |
| Mutant vorwärts (f_{M}) | GGACTTGGAGGACTCTGTGATG | 57,5 | 101 |
| Mutant rückwärts (r_{M}) | AGTAAGGCCCCTCTGCTCCCCTTACT | 66,3 | |

11. Verfahren nach Anspruch 10, wobei die Segmente an den linken 5'-Enden der Sequenzen der Wild-Typ inneren Primer (f_{WT}) und mutanten inneren Primer (r_{M}) die Sequenzen sind, die hinzugefügt wurden, um die Haarnadelstrukturen zu erhalten.

12. Verfahren nach Anspruch 11, wobei die einzelne Polymerase-Kettenreaktion (PCR) mittels eines Reaktionsgemisches durchgeführt wird, das enthält:
- Puffer: 1 x
- Mg++: 2mM
- dNTP (Deoxiribonukleosidtriphosphate): jeweils 250 mM
- Mutant vorwärts Primer: 400 nM
- Mutant rückwärts Primer: 300 nM
- Wild-Typ vorwärts Primer: 200 nM
- Wild-Typ rückwärts Primer: 150 nM
- Taq Polymerase-Heißstarter: 1 Einheit
- Wasser für Molekularbiologie: 23 µl
- Genome DNA: 2 µl.

13. Verfahren nach Anspruch 12, wobei die kritische Temperatur (Tc) des Produkts (f_{M}r_{WT}), das von den beiden äußeren Primern (f_{M}, r_{WT}) erzeugt wird und eine Länge von 233 bp hat, 87,9 °C beträgt.

14. Verfahren nach Anspruch 12 oder 13, wobei das Vervielfältigungsprotokoll von einem Thermocycler durchgeführt wird mittels: anfänglichem Schmelzen für 3 Minuten bei 95 °C, dann 40 Schmelzzyklen bei 87,7 °C für 30 Sekunden, Anlassen bei 67 °C für 15 Sekunden und Extension bei 72 °C für 15 Sekunden.

15. Verfahren nach Anspruch 14, wobei die Temperatur, bei welcher die Schmelzzyklen stattfinden um 0,2 °C niedriger als die Tc des Produkts (f_{M}r_{WT}) ist, das von den beiden äußeren Primern (f_{M}, r_{WT}) erzeugt wird.

## Revendications

1. Procédé d'identification de variations de séquence nucléotidique, comprenant les étapes de :
- amplification d'un échantillon d'ADN, comprenant le site d'une variation à identifier, au moyen d'une réaction en chaîne par polymérase (PCR) simple avec l'utilisation simultanée de quatre amorces différentes comprenant :
- une amorce interne (f_{wT}) spécifique de l'allèle de type sauvage, ayant une base complémentaire de la base correspondante de l'allèle de type sauvage à son extrémité 3' et étant complémentaire et anti-parallèle du brin d'ADN négatif ou antisens (AS) ;
- une amorce externe (f_{M}) non spécifique d'un allèle, complémentaire et anti-parallèle d'une région dans le brin négatif ou le brin antisens (AS) à une position située en 3' de l'amorce (f_{wT}), sur le même brin ;
- une amorce interne (r_{M}), spécifique de l'allèle mutant, ayant une base complémentaire de la base correspondante de l'allèle mutant à son extrémité 3' et étant complémentaire et anti-parallèle du brin d'ADN positif ou sens (S) ;
- une amorce externe (r_{WT}) non spécifique d'un allèle, complémentaire et anti-parallèle d'une région dans le brin positif ou le brin d'ADN sens (S) à une position située en 3' de l'amorce (r_{M}) sur le même brin ;
- obtention pour chaque copie de la séquence, d'un produit (wt) en présence de l'allèle de type sauvage et d'un produit (mut) en présence de l'allèle mutant dans lesquels :
- les deux produits sont différenciés par la taille, la température de fusion (Tm) et la température critique (Tc) - où « température critique » désigne la température de fusion au-dessous de laquelle une cible de PCR spécifique n'est pas amplifiée ;
- la détection des produits de réaction de type sauvage (wt) et mutant (mut) sur la base desdites caractéristiques de différenciation au moyen d'une séparation électrophorétique sur un gel d'agarose, ou par détermination de la température de fusion spécifique au moyen de l'analyse de la courbe de fusion en cas de PCR en temps réel,
**caractérisé en ce que** le procédé comprend en outre l'étape de :
- prévention de l'apparition du produit généré par les deux amorces externes, au moyen d'une étape de fusion réalisée à une température inférieure à la température de fusion critique (Tc) du produit généré par l'amorçage avec la combinaison des deux amorces externes, et supérieure à la température critique (Tc) caractéristique des deux autres produits générés par les amorces (f_{wT}) et (r_{WT}) et par les amorces (f_{M}) et (r_{M}), de sorte que seuls ces deux produits (wt) et (mut) Sont présents à la fin de la réaction de PCR.

2. Procédé selon la revendication 1, dans lequel la température de ladite étape de fusion est inférieure de quelques dixièmes de degré à la température critique dudit produit généré par l'amorçage avec la combinaison des deux amorces externes (f_{M}, r_{WT}).

3. Procédé selon la revendication 1 ou 2, dans lequel les amorces sont choisies parmi les amorces habituellement employées dans des procédés de génotypage fondés sur une PCR avec une extension d'amorce spécifique d'un allèle, en particulier des amorces en épingle à cheveux et des amorces modifiées par un acide nucléique bloqué.

4. Procédé selon la revendication 3, dans lequel les amorces spécifiques d'un allèle sont des amorces en épingle à cheveux ayant, sans la queue nucléotidique provenant de la structure en épingle à cheveux, une longueur dans la plage de 18 à 25 nucléotides et une température de fusion Tm dans la plage de 55 °C à 60 °C, et la queue nucléotidique a une longueur dans la plage de 5 à 7 nucléotides avec une enthalpie, calculée en tant que -ΔG de la structure en épingle à cheveux, dans la plage de -4,2 à -7,0 à température ambiante et dans des solutions tampons de PCR standard.

5. Procédé selon l'une quelconque des revendications précédentes, dans lequel ladite étape de détection est réalisée par séparation électrophorétique sur un gel ou dans un tube capillaire ou par l'analyse de courbes de fusion standard ou haute résolution.

6. Procédé selon l'une quelconque des revendications 1 à 4, dans lequel ladite étape de détection est réalisée sur un support solide.

7. Procédé selon l'une quelconque des revendications précédentes, utilisé pour des tests génétiques de diagnostic.

8. Procédé selon la revendication 7, dans lequel les tests de diagnostic sont des tests visant à identifier le polymorphisme responsable de la maladie génétique de von Willebrand de type I chez le chien.

9. Procédé selon l'une quelconque des revendications 1 à 6, dans lequel les amorces internes sont des amorces spécifiques des variations résultant de polymorphismes obtenus après une réaction au bisulfite dans une analyse de méthylation de l'ADN.

10. Procédé selon la revendication 8, dans lequel les quatre amorces sont conçues en utilisant les séquences génomiques de référence du facteur de von Willebrand chez le chien et dans lequel les séquences des amorces et leurs caractéristiques thermodynamiques sont comme suit :
| Amorce | Séquence | r | longueur du produit (pb) |
|---|---|---|---|
| Sens type sauvage (f_{wT}) | **CGACAGTGTGAGGACAACTGCCTGTCG** | 67,4 | 184 |
| Inverse type sauvage (r_{WT}) | **GCTGCCTTTCACCCAACCTC** | 58,5 | |
| Sens mutante (f_{M}) | **GGACTTGGAGGACTCTGTGATG** | 57,5 | 101 |
| Inverse mutante (r_{M}) | **AGTAAGGCCCCTCTGCTCCCCTTACT** | 66,3 | |

11. Procédé selon la revendication 10, dans lequel les segments à l'extrémité gauche 5' des séquences de l'amorce interne de type sauvage (f_{wT}) et de l'amorce interne mutante (r_{M}) sont les queues ajoutées pour obtenir une structure en épingle à cheveux.

12. Procédé selon la revendication 11, dans lequel la réaction en chaîne par polymérase (PCR) simple est réalisée au moyen d'un mélange réactionnel comprenant :
- Tampon : 1x
- Mg++ : 2 mM
- dNTP (désoxyribonucléoside triphosphates) : 250 mM chacun
- amorce sens mutante : 400 nM
- amorce inverse mutante : 300 nM
- amorce sens de type sauvage : 200 nM
- amorce inverse de type sauvage : 150 nM
- Taq polymérase Hot Start : 1 unité
- eau pour biologie moléculaire : 23 µl
- ADN génomique : 2 µl.

13. Procédé selon la revendication 12, dans lequel la température critique (Tc) du produit (f_{M}r_{WT}) généré par les amorces externes (f_{M}, r_{WT}), ayant une longueur de 233 pb, est 87,9 °C.

14. Procédé selon la revendication 12 ou 13, dans lequel le protocole d'amplification est réalisé sur un thermocycleur au moyen de : fusion initiale pendant 3 minutes à 95 °C, puis 40 cycles de fusion à 87,7 °C pendant 30 secondes, annelage à 67 °C pendant 15 secondes et extension à 72 °C pendant 15 secondes.

15. Procédé selon la revendication 14, dans lequel la température à laquelle les cycles de fusion ont lieu est inférieure de 0,2 °C à la Tc du produit (f_{M}r_{WT}) généré par les amorces externes (f_{M}, r_{WT}).
